# EUROPEAN PATENT APPLICATION

(11) **EP 2 324 827 A1**
(43) Date of publication of application: **25.05.2011**
(21) Application number: 09808321.5
(22) Date of filing: 21.08.2009
(51) Int. Cl.: A61K 31/202, A61K 31/232, A61K 31/616, A61P 11/00, A61P 13/12, A61P 43/00

(54) **THERAPEUTIC AGENT FOR ANCA-RELATED VASCULITIS**

(30) Priority: 22.08.2008 JP 2008214707
(71) Applicant: Mochida Pharmaceutical Co., Ltd., Tokyo 160-8515 (JP); The University of Tokyo, Bunkyo-Ku Tokyo 113-8654 (JP)
(72) Inventor: HIRAHASHI Junichi, Tokyo 113-8654 (JP); ARITA Makoto, Tokyo 113-8654 (JP); HISHIKAWA Keiichi, Tokyo 113-8654 (JP); FUJITA Toshiro, Tokyo 113-8654 (JP); NAKAKUKI Masanori, Tokyo 160-8515 (JP)
(74) Representative: Grünecker, Kinkeldey, Stockmair & Schwanhäusser Anwaltssozietät
(86) International application number: PCT/JP2009/064669
(87) International publication number: WO 2010/021385

(57) **Abstract**

Disclosed is an agent for preventing/treating ANCA-related vasculitis and/or preventing the recurrence of ANCA-related vasculitis. Also disclosed is a therapeutic method using the agent. Specifically disclosed is a pharmaceutical composition comprising at least one member selected from the group consisting of eicosapentaenoic acid, a pharmaceutically acceptable salt thereof and an ester thereof as an active ingredient. The pharmaceutical composition is useful for the prevention of recurrence of ANCA-related vasculitis, the treatment of chronic ANCA-related vasculitis, and the prevention and treatment of rapidly progressive glomerulonephritis (RPGN).

## Description

### TECHNICAL FIELD

The present invention relates to prophylactic or therapeutic, and/or relapse-suppressing agents for diseases associated with anti-neutrophil cytoplasmic antibodies (ANCAs), ANCA-associated vasculitis in particular, and therapeutic methods using such agents.

### BACKGROUND ART

Anti-neutrophil cytoplasmic antibodies (ANCAs) are the autoantibody of the IgG type to the cytoplasm of neutrophil that have recently proved very likely to be found in cases presenting small-vessel vasculitis chiefly in the kidney or lung. ANCAs are divided into two classes based on the pattern of fluorescent staining, that is to say, into cytoplasmic ANCAs (C-ANCAs) and perinuclear ANCAs (P-ANCAs), whereupon typical antigens are proteinase-3 (PR-3) corresponding to C-ANCAs and myeloperoxidase (MPO) corresponding to P-ANCAs.

ANCA-associated vasculitis is the pathologic condition in which ANCAs are detected in the serum, and which is very likely to be involved in rapidly progressive glomerulonephritis (RPGN) due to necrotic or granulomatous, microvascular and capillary vasculitides in the kidney or lung, or in a pulmonary-renal syndrome with hematurina, a chronic nephritic syndrome, or a pulmonary finding (pulmonary hemorrhage, interstitial pneumonia). C-ANCAs are very likely to be found in Wegener's granulomatosis, while P-ANCAs are often found in microscopic polyangiitis, Churg-Strauss syndrome (allergic granulomatous angiitis), and so forth.

During the treatment of ANCA-associated vasculitis, the vasculitis activity is systemically evaluated, and the treatment is performed stepwise by initial remission induction therapy, remission maintenance (relapse suppression) therapy, and relapse therapy. In each therapeutic phase, holding down of the ANCA level is required.
Cases of acute ANCA-associated vasculitides in which the ANCA titer is high, and RPGN, pulmonary hemorrhage, and so forth are recognized are subjected to plasma exchange, use of a corticosteroid drug in high doses, immunosuppressive therapy chiefly with cyclophosphamide, or the like as an initial remission induction therapy. Even after the disease activity decreases and the ANCA titer is reduced, administration of a steroid drug or an immunosuppressant such as cyclophosphamide is continued, with the dose being modified, as a remission maintenance therapy because ANCA-associated vasculitis is liable to recur. The therapy to be performed as a relapse therapy is selected according to the initial remission induction therapy.

Cases of chronic ANCA-associated vasculitides in which the ANCA titer is relatively low, and a chronic nephritis or chronic renal failure as a renal symptom or pulmonary fibrosis as a pulmonary symptom is presented are subjected to a treatment with a moderate corticosteroid administered alone or in combination with a low dose of an immunosuppressant (see Non-Patent Literature 1).

As described above, drugs capable of holding down the ANCA titer, such as steroids and immunosuppressants, are used for the treatment of ANCA-associated vasculitis. A long-term use of such drugs may increase the risk of developing an infectious disease. Infectious diseases may trigger ANCA-positive conversion, and cause severe conditions particularly in those subjects who are liable to be immunocompromised, such as elderly persons and tumor-bearing patients.
Such a drug capable of holding down the ANCA titer is hitherto unknown that can be used as a fundamental therapeutic agent for ANCA-associated vasculitis over a long period of time particularly in remission maintenance (relapse suppression) therapy requiring a long-term use of the drug, even if the subject to be treated is immunocompromised or has an infectious disease as a complication, and consequently suppress the relapse of ANCA-associated vasculitis, and a novel therapy has eagerly be awaited.

Medicaments for the prevention/treatment of ANCA-associated vasculitis have already been proposed. For instance, a method of treating ANCA-associated vasculitis by administering the antagonist which binds to a B-cell surface marker, such as CD20 antibody (see Patent Literature 1), diacylglycerol as a medicament for preventing and/or treating vasculitis syndromes, such as ANCA-associated arthritis, by reducing the Mac-1 or VCAM-1 expression level to suppress inflammation due to macrophage infiltration or vascular endotherial inflammation (see Patent Literature 2), and a combined application of a benzimidazole drug known for its effect of inhibiting NF-κB associated with inflammation and an angiotensin converting enzyme inhibitor, angiotensin II receptpr antagonist, interferon or the like (see Patent Literature 3) have been proposed.

On the other hand, ethyl icosapentate ester (hereafter referred to as "EPA-E") is clinically finding wide application as an medicament for ameliorating ulcer, pain and cold sense attendant on arteriosclerosis obliterans, or a therapeutic agent for hyperlipidemia. Known mechanisms of the ester include those for lipid metabolism improvement, amelioration of vascular endothelial dysfunction and stabilization of plaques, as well as for thrombogenesis inhibition by the inhibition of platelet aggregation.

It is generally possible to give anticoagulant or antiplatelet therapy concurrently with the administration of a steroid or immunosuppressant. There is a report on one case in which the subject having ANCA-associated nephritis complicated with IgA nephropathy, who had received steroid pulse therapy, was caused to take 35 mg of predonisolone and, concurrently, receive antiplatelet therapy with eicosapentaenoic acid and dipyridamole (see Non-Patent Literature 2). It has recently been reported that one-time abdominal administration of highly pure EPA suppressed the rejection reaction in a recipient mouse having the heart transplanted from a heterologous donor mouse (see Non-Patent Literature 3). According to the report, it was confirmed that the highly pure EPA induced regulatory T cell production through the activation of PPAR-γ, and suppressed CD80 and CD86 having been increased in expression in dendritic cells of the spleen. EPA, however, has never been used in remission maintenance (relapse suppression) therapy for ANCA-associated vasculitis and, as having been unknown for its ANCA titer-reducing effect, has not been used either as a fundamental therapeutic agent for ANCA-associated vasculitis taking advantage of such medicinal efficacy.

### CITATION LIST

### PATENT LITERATURE

- Patent Literature 1:: JP 2008-515890 A
- Patent Literature 2:: JP 2007-119387 A
- Patent Literature 3:: JP 2005-281283 A

### NON-PATENT LITERATURE

Non-Patent Literature 1: Mebio, Vol. 20, No. 10, pp. 21-27, October, 2003.
Non-Patent Literature 2: KIDNEY AND DIALYSIS, Vol. 59, No. 3, pp. 563-566, September, 2005.
Non-Patent Literature 3: American Journal of Transplantation, Volume 9, Issue 6, pages 1294-1307, published online: 13 May, 2009.

### SUMMARY OF INVENTION

### TECHNICAL PROBLEMS

An object of the present invention is to find a fundamental therapeutic agent for ANCA-associated vasculitis suppressing the ANCA activity, particularly suitable for the remission maintenance (relapse suppression) therapy which is to be performed over a long period of time, and a therapeutic method using such an agent.
Another object of the present invention is to solve the problems with the conventional ANCA reduction therapy, such as the use of a steroid or immunosuppressant, so as to provide the agent for suppressing ANCA production which has fewer side effects and is safely applicable to a wide variety of subjects, and a therapeutic method using such an agent.

### SOLUTION TO PROBLEMS

The inventors of the present invention concentrated on researches in order to achieve the above objects, and found at last that EPA has a prominent effect of suppressing ANCA production in an ANCA-associated vasculitis model animal, which makes it possible to fundamentally treat ANCA-associated vasculitis. It was thus proved that EPA improves the survival rate of an ANCA-associated vasculitis model animal.
In addition, analyses at the genetic level (Foxp3, PD-1, PD-L1, PD-L2, CTLA4) having been conducted on various organs of model animals suggested the possibility that EPA has a suppressive effect on excessive autoimmunity in the kidney in particular.
As a result, the inventors found that EPA, as having an ANCA production-suppressing effect directly exerted on ANCA-associated vasculitis and an autoimmunity-regulating effect in combination, is particularly suitable for the remission maintenance (relapse suppression) in a subject after the remission of acute phase symptoms of ANCA-associated vasculitis that requires a long-term use of a drug, so as to complete the present invention.

The present invention provides the pharmaceutical composition as described below.
In one embodiment, the pharmaceutical composition according to the present invention is the pharmaceutical composition for suppressing the relapse of ANCA-associated vasculitis that contains as an active ingredient at least one selected from the group consisting of icosapentaenoic acid as well as pharmaceutically acceptable salts and esters thereof.
The pharmaceutical composition as above is preferably administered to the subject who has finished receiving remission induction therapy for ANCA-associated vasculitis.

In another embodiment, the pharmaceutical composition according to the present invention is the pharmaceutical composition for treating a chronic ANCA-associated vasculitis that contains as an active ingredient at least one selected from the group consisting of icosapentaenoic acid as well as pharmaceutically acceptable salts and esters thereof.
Patients with chronic ANCA-associated vasculitides and subjects after the remission of acute phase symptoms of ANCA-associated vasculitis may be negative for, or low in level of, the ANCA titer as described later.

In a preferred embodiment, the pharmaceutical composition of the present invention is administered to a subject with impaired renal function.
In another preferred embodiment, the pharmaceutical composition of the present invention is administered to a subject having rapidly progressive glomerulonephritis (RPGN).
In yet another preferred embodiment, the pharmaceutical composition of the present invention is applied in combination with aspirin.

In another embodiment, the pharmaceutical composition according to the present invention is the pharmaceutical composition for preventing rapidly progressive glomerulonephritis (RPGN) that contains as an active ingredient at least one selected from the group consisting of icosapentaenoic acid as well as pharmaceutically acceptable salts and esters thereof, and is administered to an ANCA-positive subject.
Specifically, the pharmaceutical composition and therapeutic method of the present invention are as follows.

(1) A pharmaceutical composition for preventing or treating ANCA-associated vasculitis/suppressing relapse of ANCA-associated vasculitis in a subject, which contains as an active ingredient at least one selected from the group consisting of icosapentaenoic acid as well as pharmaceutically acceptable salts and esters thereof.
(2) The pharmaceutical composition according to (1) as above, wherein the subject is a subject who is ANCA-positive and/or has a high ANCA titer.
(3) The pharmaceutical composition according to (1) or (2) as above, which is used for suppressing ANCA production in the subject, and/or reducing ANCA in the subject, and/or keeping the subject low in ANCA level or ANCA-negative.

(4) The pharmaceutical composition according to any one of (1) through (3) as above, wherein the subject is a subject who has finished receiving remission induction therapy for ANCA-associated vasculitis.
(5) The pharmaceutical composition according to (4) as above, wherein the subject is a subject who does not become negative for ANCA after the reception of remission induction therapy, or who has become negative for ANCA after the reception of remission induction therapy, but will soon return positive for ANCA.
(6) The pharmaceutical composition according to any one of (1) through (5) as above, wherein the subject is a subject who is ANCA-positive and/or has a high ANCA titer, and to whom a corticosteroid drug is administered.
(7) The pharmaceutical composition according to (6) as above, which is used for early reduction of the corticosteroid drug in dose and/or early discontinuation of the corticosteroid drug.
(8) The pharmaceutical composition according to any one of (1) through (7) as above, wherein the subject is a subject with impaired renal function.
(9) The pharmaceutical composition according to any one of (1) through (8) as above, wherein the subject is a subject who has a chronic ANCA-associated vasculitis.
(10) The pharmaceutical composition according to any one of (1) through (9) as above, wherein the subject is a subject with a history of rapidly progressive glomerulonephritis (RPGN).

(11) The pharmaceutical composition according to any one of (1) through (8) as above, wherein the subject is a subject who is ANCA-positive but asymptomatic.
(12) The pharmaceutical composition according to any one of (1) through (11) as above, which is administered for the prevention of rapidly progressive glomerulonephritis (RPGN).
(13) The pharmaceutical composition according to any one of (1) through (12) as above, the subject is a subject highly liable to develop ANCA-associated vasculitis.

(14) The pharmaceutical composition according to any one of (1) through (13) as above, the subject is a subject who meets one or more conditions selected from the group consisting of being immunocompromised, having an infectious disease as a complication, being elderly, being a tumor-bearing patient, having a history of tuberculosis, receiving no steroid drugs, and receiving no immunosuppressants.
(15) The pharmaceutical composition according to any one of (1) through (14) as above, the subject is a subject in whom regulatory T cells have been reduced in number and/or function.
(16) The pharmaceutical composition according to any one of (1) through (15) as above, the subject is a subject in whom CD80 and/or CD86 on antigen-presenting cells has been increased in expression.
(17) The pharmaceutical composition according to any one of (1) through (16) as above, which is administered for six months or longer.
(18) The pharmaceutical composition according to any one of (1) through (17) as above, which is applied in combination with aspirin.
(19) The pharmaceutical composition according to any one of (1) through (18) as above, which is applied in combination with a PPARγ agonist.

The present invention also provides a method of preventing or treating ANCA-associated vasculitis or suppressing the recurrence of ANCA-associated vasculitis by using the pharmaceutical composition of the present invention. To be more specific:
(20) A method of preventing or treating ANCA-associated vasculitis or suppressing the relapse of ANCA-associated vasculitis, which includes administering to a subject at least one selected from the group consisting of icosapentaenoic acid as well as pharmaceutically acceptable salts and esters thereof.
(21) A method of suppressing ANCA production in a subject, and/or keeping a subject ANCA-negative or low in ANCA level, and/or reducing ANCA in an ANCA-positive subject, which includes administering to a subject at least one selected from the group consisting of icosapentaenoic acid as well as pharmaceutically acceptable salts and esters thereof.
(22) The method of preventing or treating ANCA-associated vasculitis or suppressing the relapse of ANCA-associated vasculitis according to (20) or (21) as above, wherein the subject is a subject who is ANCA-positive and/or has a high ANCA titer, and to whom a corticosteroid drug is administered, and the method is used for early reduction of the corticosteroid drug in dose and/or early discontinuation of the corticosteroid drug.
(23) A method of early reducing a corticosteroid drug in dose and/or early discontinuation of a corticosteroid drug, including administering at least one selected from the group consisting of icosapentaenoic acid as well as pharmaceutically acceptable salts and esters thereof to a subject who is ANCA-positive and/or has a high ANCA titer, and to whom the corticosteroid drug is administered.

The present invention also provides the following.
(24) Use of at least one selected from the group consisting of icosapentaenoic acid as well as pharmaceutically acceptable salts and esters thereof for the manufacture of a medicament for preventing or treating ANCA-associated vasculitis/suppressing the relapse of ANCA-associated vasculitis in a subject.
(25) At least one selected from the group consisting of icosapentaenoic acid as well as pharmaceutically acceptable salts and esters thereof for preventing or treating ANCA-associated vasculitis/suppressing the relapse of ANCA-associated vasculitis in a subject.

(26) An ANCA production suppressant, containing as an active ingredient at least one selected from the group consisting of icosapentaenoic acid as well as pharmaceutically acceptable salts and esters thereof.
(27) An ANCA-reducing agent, containing as an active ingredient at least one selected from the group consisting of icosapentaenoic acid as well as pharmaceutically acceptable salts and esters thereof.

### ADVANTAGEOUS EFFECTS OF INVENTION

According to the present invention, the therapeutic agent and therapeutic method for ANCA-associated vasculitis are provided which are applicable to a wide variety of subjects including those immunocompromised, such as elderly persons and tumor-bearing patients, those having an infectious disease as a complication of ANCA-associated vasculitis, and those with a history of tuberculosis, and which have fewer side effects even if used over a long period of time. The pharmaceutical composition of the present invention is particularly useful as a relapse-suppressing agent for a subject after the remission of acute phase symptoms of ANCA-associated vasculitis and a therapeutic agent for a chronic ANCA-associated vasculitis, with both agents being to be used over a long period of time, as well as a prophylactic agent for ANCA-associated vasculitis.
The pharmaceutical composition of the present invention will particularly be effective if applied in combination with aspirin.

### BRIEF DESCRIPTION OF DRAWINGS

Fig. 1 shows the test results concerning the action of EPA on the survival rate of SCG/Kj mice as an ANCA model.
Fig. 2 shows the test results concerning the action of EPA on the ANCA production in SCG/Kj mice.
Fig. 3 shows the evaluation results concerning the mRNA expression levels of PD-1, FD-L1, PD-L2, Foxp3, and CTLA4 in the kidneys of SCG/Kj mice.
Fig. 4 shows the evaluation results concerning the mRNA expression levels of PD-1, PD-L1, PD-L2, Foxp3, and CTLA4 in the spleens of SCG/Kj mice.

### DESCRIPTION OF EMBODIMENTS

The present invention is detailed in the following.
According to a first aspect of the present invention, there is provided a pharmaceutical composition for preventing or treating ANCA-associated vasculitis or suppressing the relapse of ANCA-associated vasculitis which contains as an active ingredient at least one selected from the group consisting of icosapentaenoic acid as well as pharmaceutically acceptable salts and esters thereof.
The term "icosapentaenoic acid" means all-cis-5,8,11,14,17-icosapentaenoic acid. Unless otherwise specified, the term "EPA" used herein means not only icosapentaenoic acid in itself but a pharmaceutically acceptable salt or derivative thereof, with the derivative being exemplified by ester, amide, phospholipid, and glyceride. While any substance denoted by "EPA" is usable as an active ingredient of the pharmaceutical composition of the present invention, "ethyl icosapentate ester (hereafter referred to as "EPA-E")" is particularly desirable as an active ingredient of the pharmaceutical composition of the present invention.

The ratio of the EPA content to the total fatty acid content of the inventive pharmaceutical composition or the dose of EPA is not particularly limited as long as the effects of the present invention are realized. EPA is preferably of high purity, that is to say, as an example, the ratio of the EPA content to the total content of the fatty acids and their derivatives in the composition is preferably not less than 40% by weight, more preferably not less than 90% by weight, and even more preferably not less than 96.5% by weight. EPADEL^{™} and EPADEL S^{™} (trade names; both manufactured by MOCHIDA PHARMACEUTICAL CO., LTD.) commercially available in Japan as a therapeutic agent for arteriosclerosis obliterans and hyperlipidemia are high purity EPA-E-containing soft capsules with a ratio of the EPA-E content to the total fatty acid content of not less than 96.5% by weight. These agents may be used in the pharmaceutical composition of the present invention.

ANCA-associated vasculitis is the pathologic condition in which anti-neutrophil cytoplasmic autoantibodies (ANCAs) to the cytoplasm of neutrophils are detected in the serum, and which is very likely to be involved in rapidly progressive glomerulonephritis (RPGN) due to necrotic or granulomatous, microvascular and capillary vasculitides in the kidney or lung, or in a pulmonary-renal syndrome with hematurina, a chronic nephritic syndrome, or a pulmonary finding (pulmonary hemorrhage, interstitial pneumonia). The ANCA-associated vasculitis which has caused nephritis is also called ANCA-associated nephritis.

In the present invention, if a subject having vasculitis is positive for one or more of four types of ANCAs, namely, C-ANCA and P-ANCA measured by an indirect fluorescent antibody technique as well as PR-3 ANCA and MPO-ANCA measured by an ELISA technique, the vasculitis is considered as an ANCA-associated vasculitis.
In the present invention, "being ANCA-positive" refers to having an ANCA titer beyond the reference range and, on the other hand, "being ANCA-negative" refers to having an ANCA titer within the reference range. The reference level may vary with criterion for diagnosis, medical facility, or measuring technique, so that it is not particularly limited. In terms of MPO-ANCA measured by ELISA, for instance, less than 10 IU/mL of ANCA is considered as indicative of being negative, and not less than 10 IU/mL of ANCA is considered as indicative of being positive. In the present invention, the unit of ANCA measurement is not particularly limited, and ANCAs may also be measured in EU or U/mL.

The pharmaceutical composition of the present invention is effective in a non-limitative manner against:
(1) vasculitis or nephritis of idiopathic type (kidney-localized type) and (2) Wegener's granulomatosis (WG) as a vasculitis or nephritis associated chiefly with C-ANCA or PR-3 ANCA; and
(1) vasculitis or nephritis of idiopathic type (kidney-localized type), (2) microscopic polyangiitis (MPA), (3) Churg-Strauss syndrome (allergic glanulomatous angiitis: AGA), (4) scleroderma renal crisis without hypertension, (5) vasculitis or nephritis induced by a drug (e.g., propylthiouracil, hydralazine, Zyloric, an antirheumetic), and (6) vasculitis or nephritis induced by silicosis or a special environmental or living factor as a vasculitis or nephritis associated chiefly with P-ANCA or MPO-ANCA.

ANCA-associated vasculitis may be involved in rapidly progressive glomerulonephritis (RPGN), a chronic nephritic syndrome, or a pulmonary-renal syndrome with a pulmonary symptom (pulmonary hemorrhage, interstitial pneumonia), and the pharmaceutical composition of the present invention is also used favorably with respect to such syndromes. Rapidly progressive glomerulonephritis is the clinical nephritic syndrome in which renal hypofunction is progressed rapidly in several weeks to several months. As described later in EXAMPLES, it was suggested that the pharmaceutical composition of the present invention is effective against an autoimmune disease in a renal disease, so that the inventive composition is particularly expected to have effects on RPGN.

The pharmaceutical composition of the present invention is favorably used for recurrence suppression in the subject who developed rapidly progressive glomerulonephritis (RPGN), for instance, and has finished receiving remission induction therapy, such as steroid pulse therapy, administration of either one or both of a corticosteroid drug and cyclophosphamide, or plasma exchange.
Exemplary diseases causing rapidly progressive glomerulonephritis (RPGN) include crescentic glomerulonephritis (of anti-GBM antibody type, immune complex type, pauci-immune type, mixed type, etc.), glomerulonephritis with crescent formation (membranoproliferative glomerulonephritis, membranous nephropathy, IgA nephropathy, non-IgA mesangial proliferative glomerulonephritis, etc.), and systemic diseases (Goodpasture's syndrome, systemic erythematodes, Wegener's granulomatosis, microscopic polyangiitis, rheumatoid arthritis, malignant tumor, etc.). The pharmaceutical composition of the present invention, as being expected to have therapeutic effects on ANCA-positive subjects, is preferably applied to the subject having pauci-immune crescentic glomerulonephritis or microscopic polyangiitis who is high in ANCA positivity.

The pharmaceutical composition of the present invention can be used for preventing ANCA-associated vasculitis.
In the present invention, being used for preventing ANCA-associated vasculitis refers to being administered for the period of time during which the subject in question is ANCA-negative, or cannot be diagnosed as having ANCA-associated vasculitis because the subject is merely ANCA-positive with no other symptoms (signs).
The term "prevention" used herein means one or more selected from among suppressing, relieving, and delaying the onset of a disease or symptom as well as reducing a disease in incidence rate.
There exist subjects having no physical symptoms (e.g., inflammation, a symptom attendant on vasculitis) except for a high ANCA titer, who are highly liable to develop a severe ANCA-associated vasculitis by certain stimulation or infection, A subject having an extremely high ANCA titer will be treated aggressively by, for instance, plasma exchange. The onset of ANCA-associated vasculitis can be prevented fundamentally by administering the pharmaceutical composition of the present invention to a subject having no symptoms (signs) except for a high ANCA titer so as to reduce ANCA. In other words, in a preferred embodiment, the pharmaceutical composition of the present invention is a composition for preventing ANCA-associated vasculitis to, be applied to a subject having a high ANCA titer but asymptomatic.
In another preferred embodiment, the pharmaceutical composition of the present invention is a pharmaceutical composition for reducing ANCA in an ANCA-positive subject but asymptomatic.

Since it depends on the measuring technique used, "a high ANCA titer" is not defined in the present invention, whereupon such a titer is desirably 50 IU/mL or higher, more desirably 100 IU/mL or higher, and even more desirably 200 IU/mL or higher, especially 500 IU/mL or higher, for MPO-ANCA.

The pharmaceutical composition of the present invention is favorably applied to a human highly liable to develop ANCA-associated vasculitis (due to a genetic factor, consumption of a drug inducing the disease, an environmental factor such as silicon, or the like), for instance, to prevent ANCA-associated vasculitis from onset. Desirably, the inventive composition prevents rapidly progressive glomerulonephritis, a chronic nephritic syndrome, a pulmonary-renal syndrome, and so forth.

The pharmaceutical composition of the present invention is administered to a subject having ANCA-associated vasculitis for the period of time during which the subject is ANCA-positive, and abnormal test results or clinical symptoms of ANCA-associated vasculitis are found in the subject. In that case, the inventive composition is desirably used in order to reduce the ANCA titer in the serum, or to suppress, relieve or delay the onset of a systemic symptom (e.g., fever, weight loss, easy fatigability), rapidly progressive glomerulonephritis (RPGN), pulmonary hemorrhage, interstitial pneumonia or a pulmonary-renal syndrome, the ischemia, infarct or hemorrhage in a tissue, and so forth.
Abnormal test results are exemplified by an elevated erythrocyte sedimentation rate, increase in acute phase reactant (CRP, SAA, alpha 2 or beta globulin fraction, fibrinogen), leucocytosis, anemia, occult hematuria, occurrence of blood casts, occurrence of urinary proteins, and increase in serum creatinine.

As an example, the pharmaceutical composition of the present invention is administered to the subject in whom the ANCA level is higher than normal, and an acute phase symptom, such as a systemic symptom (e.g., fever, weight loss, easy fatigability), rapidly progressive glomerulonephritis (RPGN), pulmonary hemorrhage, interstitial pneumonia and a pulmonary-renal syndrome, has occurred, so as to ameliorate or relieve the symptom.
The remission induction for ANCA-associated vasculitis is generally carried out by the use of a corticosteroid drug, steroid pulse therapy, immunosuppressive therapy with cyclophosphamide, plasma exchange, or the like over three to six months. The pharmaceutical composition of the present invention can additionally be used in such remission induction therapy as above, In that case, combined application of aspirin is desirable.
The pharmaceutical composition of the present invention is favorably applied to so-called intractable subjects, such as the subject who does not become negative for ANCA after the reception of remission induction therapy, and the subject with an unstable condition who becomes negative for ANCA after the reception of remission induction therapy, but soon returns positive. In the present invention, "soon returning positive for ANCA" refers to returning positive for ANCA within one year after becoming negative, and it contains the case of returning positive for ANCA within six months, three months, one month, or within ten days, for instance. The inventive composition is considered as different in action mechanism from the conventional ANCA-reducing agent, so that its application is useful as one of therapeutic variations.
In other words, in a preferred embodiment, the pharmaceutical composition according to the present invention is the pharmaceutical composition for keeping the subject who does not become negative for ANCA after the reception of remission induction therapy, or who has become negative for ANCA after the reception of remission induction therapy, but will soon return positive, low in ANCA level or ANCA-negative that contains as an active ingredient at least one selected from the group consisting of icosapentaenoic acid as well as pharmaceutically acceptable salts and esters thereof.

The pharmaceutical composition of the present invention may also be applied, preferably in order to perform a long-term treatment, to the subject having a chronic ANCA-associated vasculitis whose ANCA titer is relatively low, and who presents a chronic nephritis or chronic renal failure as a renal symptom or pulmonary fibrosis as a pulmonary symptom. In that case, a combined application of EPA-E and aspirin is particularly desirable.

The pharmaceutical composition of the present invention can be used in remission maintenance (relapse suppression) therapy performed after the completion of remission induction therapy for ANCA-associated vasculitis. ANCA-associated vasculitis is generally liable to recur, so that it is said that remission maintenance (relapse suppression) therapy should be performed for about 5 to 7 years after the initial treatment. In other words, the pharmaceutical composition of the present invention is favorably used as a relapse-suppressing agent for ANCA-associated vasculitis. Being used as a relapse-suppressing agent for ANCA-associated vasculitis refers to being administered within a specified period of time after the completion of remission induction therapy for ANCA-associated vasculitis, desirably to being administered within the period of time after the completion of remission induction therapy during which the subject in question is ANCA-negative or low in ANCA level. In the present invention, "suppressing the relapse of ANCA-associated vasculitis" includes reducing ANCA-associated vasculitis in incidence rate, or delaying the onset of ANCA-associated vasculitis, in the subjects who have finished receiving remission induction therapy for ANCA-associated vasculitis.

In the present invention, "being low in ANCA level" refers to having an ANCA titer up to about three times as large as the reference level. If the reference level is 10 IU/mL for MPO-ANCA, for instance, the inventive composition is administered to the subject whose ANCA titer is preferably 30 IU/mL or lower, and more preferably 20 IU/mL or lower.
It is desirable that the pharmaceutical composition of the present invention has an effect of suppressing, relieving or delaying the relapse of a symptom attendant on ANCA-associated vasculitis.
In a preferred embodiment, the pharmaceutical composition according to the present invention is the pharmaceutical composition for keeping a subject ANCA-negative or low in ANCA level that contains as an active ingredient at least one selected from the group consisting of icosapentaenoic acid as well as pharmaceutically acceptable salts and esters thereof, and is administered to a subject after the reception of remission induction therapy for ANCA-associated vasculitis.

For instance, the pharmaceutical composition of the present invention is suitable for a long-term application to a subject with a weakened immune status, such as an elderly person, a tumor-bearing patient, a subject having an infectious disease as a complication, and a subject with a history of tuberculosis, as a remission maintenance (relapse suppression) therapy performed after the completion of remission induction therapy for ANCA-associated vasculitis. In the present invention, elderly persons are not particularly limited in age, although it is preferable to define elderly persons as being 65 years old and above in accordance with a criterion proposed by the World Health Organization (WHO) of the United Nations.
The pharmaceutical composition of the present invention is also applied favorably to such a subject as has become negative for ANCA owing to remission induction therapy, and yet is liable to return positive.

As described above, the pharmaceutical composition of the present invention for preventing or treating ANCA-associated vasculitis/suppressing the relapse of ANCA-associated vasculitis is preferably administered to an ANCA-positive subject and/or a subject having a high ANCA titer so as to suppress ANCA production in the subject, and/or reduce ANCA in the subject, and/or keep the subject low in ANCA level or ANCA-negative. It is desirable that a subject is kept low in ANCA level or ANCA-negative for a long period of time by suitably administering the pharmaceutical Composition of the present invention to the subject. The long period of time as mentioned above lasts at least six months, and is preferably one year or longer, more preferably three years or longer, and even more preferably five years or longer, especially seven years or longer.

It is suggested that the pharmaceutical composition of the present invention can induce regulatory T cells in an ANCA- model animal, in its kidney in particular, so as to participate in an autaimmunoregulatory mechanism, so that the inventive composition is favorably applied to the subject in whom regulatory T cells have been reduced in number and/or function, for instance. That regulatory T cells have been reduced in number and/or function may be determined by a comparison with the mean level for healthy people or a previous level of the subject's own.
It has been reported that regulatory T cells suppress CD80 and CD86 (Onishi et al., Proc. Natl. Acad. Sci. USA, 2008 Jul. 22; 105(29): pp. 10113-10118). CD80 and CD86 are known as costimulatory molecules on antigen-presenting cells, and their suppression may allow the regulation of excessive autoiznmunity, leading to the treatment of an autoimmune disease. The pharmaceutical composition of the present invention is favorably applied to, for instance, the subject in whom CD80 and/or CD86 has been increased (up-regulated) in expression, or the subject in whom CD80/CD86 on antigen-presenting cells present in the peripheral blood or the lymphatic tissue, such as lymph nodes and spleen, has been increased in expression as compared with healthy people, and is expected to have an effect of suppressing CD80 or CD86 as increased to regulate excessive autoimmunity. That CD80 and/or CD86 has been increased (up-regulated) in expression may be determined by a comparison with the mean level for healthy people or a previous level of the subject's own.

The pharmaceutical composition of the present invention may be used in combination with a drug used in conventional remission maintenance therapy, such as an oral corticosteroid drug, azathioprine, cyclosporin, and mizoribine. In that case, the inventive composition, as having an effect of directly reducing the ANCA titer, allows the reduction of other drugs in dose, or the discontinuation of other drugs. It is desirable to, for instance, withdraw from an immunosuppressive agent, set the dose of an oral corticosteroid drug low, or even withdraw from an oral corticosteroid drug.

The dose and dosage period of EPA used in the pharmaceutical composition or therapeutic agent of the present invention are made effective amount of dosage and dosage period to the expected action of the drug, and each modified as appropriate to the dosage form, dosage route, and frequency of administration per day of the drug, the degree of a symptom, the body weight and age of a subject, and so forth.
In the case of oral administration, 0.1 to 10 g/day, preferably 0.3 to 6 g/day, more preferably 0.6 to 4 g/day, and even more preferably 0.9 to 2.7 g/day of EPA-E, for instance, is administered at a time or in two or three portions. Whether the entire amount is administered at a time or in portions may be determined as required. A preferred daily dose is 1.8 to 2.7 g, especially 2.4 g. The dose may be reduced in response to the dose of other drugs. Administration is preferably performed during meals or after meals, with an administration just after meals (within 30 minutes after a meal) being more preferred.

The period of oral administration at the above dose is not particularly limited, lasting at least six months or one year, for instance, and is preferably two years or longer, more preferably 3.5 years or longer, and even more preferably 5 years or longer, especially 7 years or longer. It is desirable that administration be continued while a pathologic condition, biochemical index, or the like related to ANCA-associated vasculitis remains, or the subject is under the situation where the risk of the onset and/or relapse of ANCA-associated vasculitis is great. Administration may also be performed every other day or two or three days a week, for instance, or with an optional drug withdrawal period for one day to about three months, preferably about one week to one month.

Preferred examples of other fatty acids contained in the pharmaceutical composition of the present invention include ω-3 polyunsaturated fatty acids other than EPA, such as docosahexaenoic acid, docosapentaenoic acid and α-linolenic acid, as well as pharmaceutically acceptable salts and esters thereof, with ethyl dacosahexaenoate ester (hereafter referred to as "DHA-E") being particularly mentioned.
The composition ratio EPA-E/DHA-E, the ratio of the EPA-E and DHA-E (hereafter referred to as "(EPA-E + DHA-E)") content to the total content of the fatty acids in the composition, and the dose of (EPA-E + DHA-E) are not particularly limited as long as the effects of the present invention are realized. (EPA-E + DHA-E) is preferably of high purity, that is to say, as an example, the ratio of the (EPA-E + DHA-E) content to the total content of the fatty acids and their derivatives in the composition is preferably not less than 40% by weight, more preferably not less than 80% by weight, and even more preferably not less than 90% by weight.

An exemplary daily dose of (EPA-E + DHA-E) is 0.3 to 20 g/day, preferably 0.5 to 12 g/day, and more preferably 1 to 8 g/day. Doses of 0.3 to 6 g/day, 0.3 to 4 g/day, and 0.3 to 1 g/day are also preferable.

In this connection, it is desirable that any long-chain saturated fatty acid content is low, and any ω-6 fatty acid, particularly arachidonic acid, is low in content even though it is a long-chain unsaturated fatty acid, whereupon a content lower than 2% by weight, in particular lower than 1% by weight, is preferred. A commercially available formulation containing EPA-E and DHA-E, such as the soft capsules containing about 46% by weight of EPA-E and about 38% by weight of DHA-E (LOVAZA™ (from Reliant Corporation; Pronova Corporation), etc.) which are commercially available in the USA, Europe, and so forth as a therapeutic agent indicated for hypertriglyceridemia or the like, may be used in the pharmaceutical composition of the present invention.

The pharmaceutical composition for ANCA-associated vasculitis of the present invention may be administered as a compound (optionally including other constituents unremovable by purification) in itself, or combined with excipients suitably selected from among conventional carriers or media, vehicles, binders, lubricants, colorants, flavors, sterilized water or vegetable oils as required, as well as innoxious organic solvents or innoxious solubilizing agents (e.g., glycerin, propylene glycol), emulsifiers, suspending agents (e.g., Tween 80, gum arabic solution), isotonicities, pH-adjusting agents, stabilizers, soothing agents, corrigents, flavoring agents, preservatives, antioxidants, buffers, colorants, and the like, so as to prepare an appropriate medical formulation. Exemplary excipients which may be contained include lactose, partially pregelatinized starch, hydroxypropylcellulose, macrogol, tocopherol, a hydrogenated oil, a sucrose ester of fatty acid, hydroxypropylmethylcellulose, titanium oxide, talc, dimethylpolysiloxane, silicon dioxide, and carnauba wax.

Since EPA-E, DHA-E, and the like are of a highly unsaturated nature, it is particularly desirable to add an effective amount of an antioxidant, for instance, at least one selected from among butylated hydroxytoluene, butylated hydroxyanisole, propyl gallate, gallic acid, a pharmaceutically acceptable quinone, and α-tocopherol.

The dosage form of the formulation, as varying with the mode of combined application of active ingredients according to the present invention, is not particularly limited. The formulation may be administered to a subject orally, intravenously, intraarterially, by inhalation, rectally, intravaginally or externally, that is to say, as an oral formulation in the form of tablet, film-coated tablet, capsule, microcapsule, granule, fine granule, powder, oral liquid preparation, syrup, jelly, inhalant or the like, or as a parenteral formulation in the form of ointment, suppository, injection (emulsion, suspension, nonaqueous solution), solid injection to be emulsified or suspended before use, transfusion solution, external preparation such as endermic preparation, or the like. For those subjects who are able to take oral formulations, easy-to-take oral formulations are desirable, so that oral administration of the formulation as included in a capsule such as soft capsule and microcapsule, in tablet form, or in film-coated tablet form is particularly preferred. It is also possible to administrate the formulation orally as an enteric preparation or an extended release preparation, or as a jelly in the case of dialysis patients or subjects with dysphagia.

The pharmaceutical composition and therapeutic method of the present invention using EPA-E may be combined with, or applied in combination with, another drug or therapeutic method. Application in combination with another drug includes administration of EPA-E and another drug as a composite formulation containing both drugs, and administration of EPA-E and another drug as separate formulations simultaneously or separately with certain time lag.
The drug to be used in the present invention as another drug is not particularly limited, and preferred examples not impairing the effects of the present invention include corticosteroid drugs (e.g., methylprednisolone for pulse therapy, oral prednisolone), immunosuppressants (e.g., high-dose intravenous cyclophosphamide, oral cyclophosphamide, methotrexate, mizoribine), immunoglobulins, azathioprine, TNFα inhibitors (e.g., infliximab, etanercept), anti-IL-5 antibody (SCH 55700), anti-IgE antibody (omalizumab), antibiotics, and antimicrobials (e.g., trimethoprim-sulfamethoxazole combination). Also preferred are PPARγ agonists which have been reported to induce regulatory T cells. Combined application of the pharmaceutical composition of the present invention and a PPARγ agonist is expected to have a synergistic effect on the regulation of excessive autoimmunity involved in ANCA-associated vasculitis. The PPARγ agonist to be used is not particularly limited but exemplified by thiazolidinedione derivatives (e.g., pioglitazone hydrochloride, rosiglitazone maleate), and angiotensin receptor blockers (ARBs; e.g., telmisartan).

Conventionally, anticoagulant therapy (warfarin), a vasodilator (prostaglandin formulation), or an antiplatelet agent (e.g., dipyridamole) may be used with respect to vascular luminal narrowing and thrombosis. The pharmaceutical composition of the present invention has the direct effect on ANCA that none of such drugs has, and it is suggested that the inventive composition can regulate autoimmunity, so that the pharmaceutical composition of the invention is extremely useful as a fundamental therapeutic agent for ANCA-associated vasculitis.

In a preferred embodiment of the pharmaceutical composition and therapeutic method of the present invention, EPA-E and aspirin are applied in combination with each other. The dosage and administration for EPA-E is preferably as described above. The dosage and administration for aspirin is not particularly limited, and aspirin may be administered at a dose of, for instance, 50 to 200 mg/day, preferably 100 mg/day. Aspirin is commercially available under the trade name of Bayaspirin^{(R)} (Bayer Yakuhin, Ltd.), for instance. If applied in combination with aspirin, EPA exerts very prominent effects on ANCA-associated vasculitis. The combination of EPA and aspirin is easy to use because of its few side effects as compared with steroid drugs or immunosuppressants. Moreover, combined EPA-aspirin application therapy has an astonishing effect of restoring deteriorated renal function. The restoration of renal function may be confirmed by various testings through the reduction in creatinine level, for instance.

In a preferred embodiment of the pharmaceutical composition and therapeutic method of the present invention, EPA-E and oral corticosteroid are applied in combination with each other. The dosage and administration for a corticosteroid drug is not particularly limited, and a corticosteroid drug can be administered at a low dose owing to the effects of the pharmaceutical composition of the present invention. In the present invention, a low dose for a corticosteroid drug refers to a dose of not more than 20 mg/day, for instance, whereupon the dose may preferably be 15 mg/day or lower, more preferably 10 mg/day or lower, and even more preferably 5 mg/day or lower, especially 2.5 mg/day or lower.
Corticosteroid drugs have a side effect of readily causing arteriosclerosis in the subjects to whom they are administered. The pharmaceutical composition of the present invention as applied preferably in combination with aspirin is advantageously effective at suppressing the progression of arteriosclerosis due to a corticosteroid drug.

According to a second aspect of the present invention, there is provided a method of preventing or treating ANCA-associated vasculitis or preventing the relapse of ANCA-associated vasculitis by using the pharmaceutical composition of the present invention. In other words, the present invention provides a method of preventing or treating ANCA-associated vasculitis/suppressing the relapse of ANCA-associated vasculitis which includes administering to a subject at least one selected from the group consisting of icosapentaenoic acid as well as pharmaceutically acceptable salts and esters thereof.
The method of the present invention desirably suppresses ANCA production in a subject, and/or keeps a subject ANCA-negative or low in ANCA level, and/or reduces ANCA in an ANCA-positive subject. The method of the present invention is a method of keeping a subject low in ANCA level or ANCA-negative preferably for at least six months or one year, more preferably for three years or longer, and even more preferably for five years or longer, especially seven years or longer, by administering the pharmaceutical composition of the present invention to the subject.
The target subject for the prophylactic method of the present invention is preferably the subject who is ANCA-negative, or cannot be diagnosed as having ANCAs-associated vasculitis because the subject is merely ANCA-positive with no other clinical symptoms. Preferably, the target is a subject highly liable to develop ANCA-associated vasculitis (due to a genetic factor, consumption of a drug inducing the disease, an environmental factor such as exposure to silicon, or the like), or a subject determined from a renal examination, a genetic factor, or the like to be highly liable to develop a renal disease.
The dosage, administration and dosage period is as described above with respect to the first aspect of the present invention.
The inventive composition is desirably administered in order to keep a subject ANCA-negative or low in ANCA level, or to suppress, relieve or delay the onset of a clinical symptom attendant on ANCA-associated vasculitis, rapidly progressive glomerulonephritis, or a pulmonary-renal syndrome in an ANCA-positive subject.
In a preferred embodiment, the prophylactic method of the present invention is a method of reducing ANCA-associated vasculitis in incidence rate, which includes administering at least one selected from the group consisting of icosapentaenoic acid as well as pharmaceutically acceptable salts and esters thereof to the subject who is ANCA-positive but asymptomatic.

The target subject for the therapeutic method of the present invention is the subject who is ANCA-positive, and in whom an abnormal test result or a clinical symptom attendant on ANCA-associated vasculitis is found.
The dosage, administration and dosage period is not particularly limited but is as described above with respect to the first aspect of the present invention. A desirable embodiment of the inventive therapeutic method is such that, during the remission induction therapy performed on the subject who has ANCA-associated vasculitis complicated with rapidly progressive glomerulonephritis, for instance, the pharmaceutical composition of the present invention is administered as the corticosteroid drug or immunosuppressant to be administered is stepwise reduced in dose, so as to switch the therapeutic agent to the inventive composition, and continue the administration of the inventive composition until the risk of relapse disappears.
It is also preferable to administer the pharmaceutical composition of the present invention to the subject who does not become negative for ANCA even after the reception of remission induction therapy.
In other words, in a preferred embodiment, the therapeutic method of the present invention is the method of keeping a subject ANCA-negative or low in ANCA level which includes administering at least one selected from the group consisting of icosapentaenoic acid as well as pharmaceutically acceptable salts and esters thereof to the subject who does not become negative for ANCA after the reception of remission induction therapy for ANCA-associated vasculitis, or who has become negative for ANCA after the reception of remission induction therapy, but will soon return positive for ANCA.

The target subject for the relapse-suppressing method of the present invention is the subject who has finished receiving remission induction therapy for ANCA-associated vasculitis, preferably the subject who is ANCA-negative or low in ANCA level. The dosage regime is not particularly limited but is as described above with respect to the first aspect of the present invention. A desirable dosage period is substantially long, lasting one year or longer, preferably three years or longer, and more preferably five years or longer, for instance.

A drug other than EPA may be applied in combination during the suppression of relapse, for instance. The inventors of the present invention experienced the case in which a long-term application of EPA and aspirin in combination was effective for the suppression of the relapse of ANCA-associated vasculitis. The relapse-suppressing effect was achieved by administering EPA-E and aspirin after steroid pulse therapy and removal of ANCA by plasma exchange.
In a preferred embodiment, the method according to the present invention is the method of suppressing the relapse of ANCA-associated vasculitis that includes administering EPA-E and aspirin in combination to the subject after the reception of remission induction therapy, such as steroid pulse therapy and plasma exchange, who has preferably become negative for ANCA, or become negative for CRP. Steroid pulse therapy is conducted such that a steroid drug is administered intensively for a short period of time, followed by the drug withdrawal for a certain period of time. In an exemplary therapy, 500 to 1000 mg/day of methylprednisolone is administered for three or four sequential days, which is repeated every one to three weeks depending on the therapeutic effects as achieved.

Conventionally, a corticosteroid drug is often administered to a subject after the reception of remission induction therapy, with the dose being stepwise reduced from an initial dose of about 40 mg, and a dose of about 10 mg is maintained for about one to two years. According to the method of the present invention, the dose of a corticosteroid drug can be reduced at a relatively fast pace (reduced to about 5 mg in a half year, for instance), so that the inventive method is also effective as a method of early discontinuation of a corticosteroid drug. In other words, in a preferred embodiment, the method according to the present invention is the method of suppressing the relapse of ANCA-associated vasculitis that includes administering EPA-E and aspirin to the subject after the reception of remission induction therapy for ANCA-associated vasculitis to whom a corticosteroid drug is administered, so as to stepwise reduce the corticosteroid drug in dose to about 5 mg within a year, for instance. The dose of a corticosteroid drug may desirably be reduced to about 5 mg within six months, more preferably within three months.
In a preferred embodiment, the method according to the present invention is the method of early reducing a corticosteroid drug in dose and/or early withdrawing from a corticosteroid drug that includes administering at least one selected from the group consisting of icosapentaenoic acid as well as pharmaceutically acceptable salts and esters thereof to the subject who is ANCA-positive and/or has a high ANCA titer, and to whom a corticosteroid drug is administered. The reduction of a corticosteroid drug in dose is not particularly limited, while a reduced dose is preferably not more than 5 mg, more preferably not more than 3 mg, and even more preferably not more than 1 mg, especially not more than 0.5 mg. Desirably, the dose of a corticosteroid drug is stepwise reduced in a subject taking the drug, and it is desirable that the subject is eventually allowed to stop taking the corticosteroid drug.

It is preferable to administer EPA-E (at a dose of 1.8 to 2.7 g/day, for instance) and aspirin (at a dose of 50 to 100 mg, for instance) over a long period of time. The dose of aspirin may be 150 to 200 mg depending on the symptom of a subject. In that case, it is desirable to apply a low dose of corticosteroid in combination.

In an embodiment of the inventive method, it is also desirable to administer corticosteroid, an immunosuppressant, aspirin, or the like for a short period of time while a subject under treatment with the pharmaceutical composition of the present invention has a condition made worse for some reason or other.

### EXAMPLES

The present invention is illustrated in reference to the following examples, to which the present invention is in no way limited.

### (Example 1) Action of EPA on the survival rate of SCG/Kj mice as an ANCA model.

### <Method>

SCG/Kj mice (seven weeks old) as a spontaneous ANCA vasculitis/nephritis model (Nippon Kayaku Co., Ltd.) were divided into three groups comprising 6 or 7 animals each, and fed on (1) fish meal-free feed F1 (Funakoshi Co., Ltd.) (control group), (2) 5% corn oil diet (manufactured by Sigma Corporation) (corn oil group), or (3) 5% EPA-E diet (diet F1 with 5% EPA-E added thereto) (EPA group) in a free-feeding state so as to observe the survival rate.

### <Results>

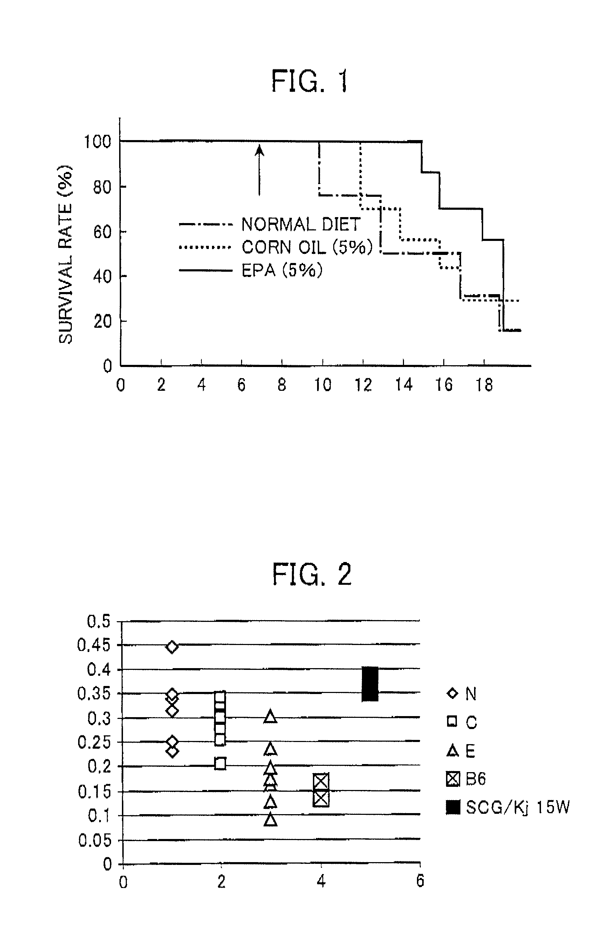

Fig. 1 shows a graph of the survival rate.
In each of the control group and the corn oil group, the survival rate began to decrease when the mice were 10 to 12 weeks old, so that corn oil was not recognized to have a survival-prolonging effect. In contract, in the EPA group, the decrease in survival rate began to be observed when the mice were about 15 weeks old, which showed that EPA-E has a survival-prolonging effect.

### (Example 2) Action of EPA on the ANCA production in SCG/Kj mice

### <Method>

SCG/Kj mice (seven weeks old) were divided into three groups comprising 6 or 7 animals each, and fed under the same conditions as Example 1 until they completed their 11th week of age. Groups of B6 mice (two animals) and of SCG/Kj mice (15 weeks old; three animals) were further provided, and the five groups were subjected to the absorbance measurement for MPO-ANCA in the serum by an ELISA technique.
The MPO-ANCA titer was measured by following a method having already been reported (Ishida-Okawara, A., Ito-Ihara, T., Muso, E. et al., Neutrophil contribution to the crescentic glomerulonephritis in SCG/Kj mice, Nephrol. Dial. Transplant. 2004; 19: 1708-1715). To put it briefly: Recombinant mouse MPO was coated onto an ELISA plate (Toyoshima Co., Tokyo, Japan) at 4°C overnight. The plate was blocked with 1% bovine serum albumin (BSA) (Sigma), then incubated at room temperature for 1.5 hours in the presence of mouse serum (50-fold dilution).
Bound MPO-ANCA was detected after an incubation for two hours in the presence of an alkaline phosphatase-labeled anti-mouse IgG antibody (Cappel). Subsequently, the secondary antibody as bound was quantified by measuring the absorbance at 405 nm after an incubation in the presence of 1 mg/ml of p-nitrophenyl phosphate (Sigma).

### <Results>

The results are shown in Fig. 2.

In the figure:
N denotes the SCG/Kj mice of the control group fed on (1) fish meal-free feed;
C denotes the SCG/Kj mice of the corn oil group fed on (2) 5% corn oil diet;
E denotes the SCG/Kj mice of the EPA group fed on (3) 5% EPA-E diet;
B6 denotes the B6 mice fed on normal diet; and
SCG/Kj denotes the SCG/Kj mice fed on normal diet.
ANCA production was evidently suppressed in the EPA group as compared with the control group and the corn oil group. The ANCA levels as measured in the EPA group were so reduced as to be comparable to those of the normal mice (B6), which showed that EPA-E has an ANCA production-suppressing effect.

### (Example 3) Evaluation of the mRNA expression levels of PD-1, PD-L1, PD-L2, Foxp3, and CTLA4 in the kidneys, lungs and spleens of SCG/Kj mice.

### <Method>

SCG/Kj mice (seven weeks old) were divided into three groups comprising 6 or 7 animals each, and fed under the same conditions as Example 1 until they completed their 11th week of age. The mRNA expression levels of PD-1, PD-L1, PD-L2, Foxp3, and CTLA4 in the kidneys, lungs and spleens of the mice were devaluated.
A frozen kidney was homogenized in a TRIzol^{(R)} reagent (Invitrogen, Carlsbad, CA). Total RNA was prepared from the homogenate, and purified using PureLink Micro-to-Midi Total RNA Purification System (Invitrogen). SuperScript™ III first-strand synthesis system (Invitrogen) was used to prepare cDNA from the purified total RNA, and quantitative real-time PCR was conducted with ABI Prism^{(R)} 7000 (Applied Biosystems, Foster City, CA). For primers and probes for PCR, TaqMan^{(R)} gene expression assays (Applied Biosystems) were used.

### <Results>

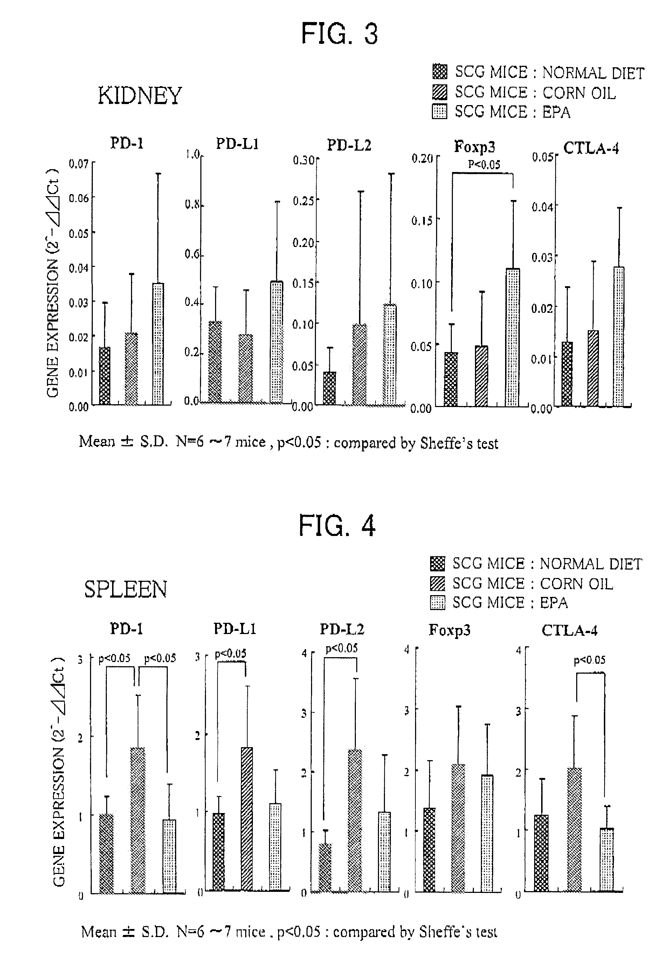

The results for the kidney and the spleen are shown in Figs. 3 and 4, respectively. In the kidney, among other organs, Foxp3 was significantly increased, and PD-1, PD-L1, PD-L2 and CTLA4 were likely to be increased in the EPA group as compared with the control group, which suggested the possibility that EPA participates in the regulatory mechanism of T cells, and has a suppressive effect on excessive autoimmunity. It was also suggested that EPA is very likely to be particularly effective in the kidney.

## Claims

1. A pharmaceutical composition for preventing or treating ANCA-associated vasculitis/suppressing relapse of ANCA-associated vasculitis, which comprises as an active ingredient at least one selected from the group consisting of icosapentaenoic acid as well as pharmaceutically acceptable salts and esters thereof, and suppresses ANCA production in a subject.

2. The pharmaceutical composition according to claim 1, wherein said subject is a subject who has finished receiving remission induction therapy for ANCA-associated vasculitis.

3. The pharmaceutical composition according to claim 1 or 2, wherein said subject is a subject who does not become negative for ANCA after reception of remission induction therapy, or who has become negative for ANCA after reception of remission induction therapy, but will soon return positive for ANCA.

4. The pharmaceutical composition according to any one of claims 1 through 3, wherein said subject is a subject who is ANCA-positive and/or has a high ANCA titer, and to whom a corticosteroid drug is administered, and the pharmaceutical composition is administered for early reduction of the corticosteroid drug in dose and/or early discontinuation of the corticosteroid drug.

5. The pharmaceutical composition according to any one of claims 1 through 4, wherein said subject is a subject who has a chronic ANCA-associated vasculitis.

6. The pharmaceutical composition according to any one of claims 1 through 4, wherein said subject is a subject who is ANCA-positive but asymptomatic.

7. The pharmaceutical composition according to any one of claims 1 through 6, which is applied in combination with aspirin.

8. An ANCA production suppressant, comprising as an active ingredient at least one selected from the group consisting of icosapentaenoic acid as well as pharmaceutically acceptable salts and esters thereof.
